# FASCICULE DE BREVET EUROPEEN

(11) **EP 2 890 333 B1**
(45) Date de publication et mention de la délivrance du brevet: **07.12.2016**
(21) Numéro de dépôt: 13762535.6
(22) Date de dépôt: 08.08.2013
(51) Int. Cl.: A61F 2/58, A61F 2/50, A61F 2/70, A61F 2/68, A61F 2/76

(54) **PROTHÈSE DE MAIN**
HANDPROTHESE
HAND PROSTHESIS

(30) Priorité: 29.08.2012 FR 1258071
(43) Date de publication de la demande: 08.07.2015
(73) Titulaire: Feltrin, Oscar, 47825 Torriana (RN) (IT); Bernhardt, Adeline, 47825 Torriana (RN) (IT)
(72) Inventeur: FELTRIN, Oscar, 47825 Torriana (RN) (IT)
(86) Numéro de dépôt international: PCT/FR2013/000211
(87) Numéro de publication internationale: WO 2014/033373

(56) Documents cités:
- FR-A- 942 172
- GB-A- 103 481
- US-A- 2 549 716
- US-A- 2 641 769
- US-A- 3 694 021
- US-A1- 2006 129 248

## Description

La présente invention concerne une prothèse de main destinée à être montée sur un moignon de bras, ou plus généralement de membre supérieur. La prothèse de main comprend de manière classique une emboiture à monter sur le moignon, un poignet, une paume de main, quatre doigts longs (index, majeur, annulaire, auriculaire) et un pouce, à l'instar d'une main naturelle. Les quatre doigts longs comprennent chacun trois phalanges mobiles articulées les unes par rapport aux autres et par rapport à la paume de main. La prothèse comprend en outre des moyens d'actionnement pour déplacer au moins certaines phalanges de chaque doigt long.

Dans le domaine des prothèses de main, on connaît les documents GB103481, FR942172 (qui divulguent tous deux une prothèse de main selon le préambule de la revendication 1), US6379393, GB237085 et US2006/224249. Le document GB237085 divulgue un système de câbles pour déplacer les doigts longs aussi bien en flexion qu'en extension. Un câble de fermeture est prévu pour fléchir les doigts et un autre câble d'ouverture est prévu pour étendre les doigts. Le document US2006/224249 prévoit un câble de flexion unique en forme de U par paire de doigts. Ainsi, un câble de flexion unique en forme de U est prévu pour fléchir l'index et le majeur, et un autre câble de flexion unique en forme de U est prévu pour fléchir l'annulaire et l'auriculaire. Pour étendre les doigts et ouvrir la main, il est prévu un système de rappel élastique. En d'autres termes, les deux câbles de flexion en forme de U travaillent en traction à l'encontre des moyens de rappel élastiques. De ce fait, il faut utiliser un moteur relativement puissant avec un frein pour surmonter la résilience des moyens élastiques de rappel et bloquer les doigts dans une position pliée ou fléchie.

Le document US2006/0129248 divulgue une autre prothèse de main comportant un système de câbles actionnés au moyen d'un arbre rotatif disposé sensiblement perpendiculairement à la direction longitudinale des doigts longs.

La présente invention a pour but de remédier aux inconvénients précités de l'art antérieur en définissant une prothèse de main électromécanique dont la flexion et l'extension des quatre doigts longs peuvent être réalisées de manière sensiblement indépendante avec un minimum d'actionneurs mécaniques (moteurs) de puissance réduite. Un autre but de la présente invention est d'éviter l'utilisation de moyens de rappel élastiques contre lesquels le ou les moteurs doivent travailler. Encore un autre but de la présente invention est d'utiliser un nombre minimum d'actionneurs (moteurs) tout en garantissant une indépendance de mouvement et de préhension des quatre doigts longs.

Pour ce faire, la présente invention propose une prothèse de main destinée à être montée sur un moignon d'un membre supérieur, la prothèse de main comprenant une emboiture à monter sur le moignon, un poignet, une paume de main, quatre doigts longs et un pouce, les quatre doigts longs comprenant chacun trois phalanges mobiles articulées les unes par rapport aux autres et par rapport à la paume, la prothèse de main comprenant en outre des moyens d'actionnement pour déplacer au moins deux des phalanges de chaque doigt long, les moyens d'actionnement comprenant deux actionneurs distincts actionnant chacun une paire de doigts longs en flexion et en extension.

En d'autres termes, les actionneurs agissent aussi bien en flexion qu'en extension, de sorte qu'ils n'ont pas besoin d'agir à l'encontre de moyens élastiques de rappel, ce qui permet de réduire considérablement la puissance, et de ce fait la taille des actionneurs. Tout comme dans le document US2006/224249, chaque actionneur agit en flexion sur une paire de doigts longs, mais en plus en extension.

Selon une forme de réalisation avantageuse, chaque paire de doigts longs comprend un câble de flexion et un câble d'extension communs à la paire de doigts longs, chaque câble de flexion et d'extension s'étendant sensiblement en forme de U sur les deux doigts de la paire de doigts longs, les deux câbles de flexion et d'extension étant sollicités en traction par un actionneur unique respectif, avantageusement avec une répartition équilibrée des forces de traction sur la paire de doigts longs. De préférence, chaque actionneur comprend un arbre rotatif disposé sensiblement perpendiculairement à la direction longitudinale des doigts longs, l'arbre comprenant au moins deux trous de passage, respectivement pour le câble de flexion et le câble d'extension, qui peuvent avantageusement coulisser à travers ces trous de passage pour équilibrer les forces de traction sur la paire de doigts longs. Du fait que les actionneurs agissent aussi bien en flexion qu'en extension permet de se passer d'un frein pour bloquer les doigts dans une position souhaitée, étant donné qu'il n'y a pas de moyen de rappel élastique qui agit à l'encontre de la traction générée par les actionneurs sur les câbles de flexion.

Selon un autre aspect intéressant de l'invention, les actionneurs peuvent actionner les phalanges proximales et moyennes des doigts longs. Ainsi, les actionneurs, par l'intermédiaire des câbles, ne déplacent que les phalanges proximales et moyennes entre elles et par rapport à la paume de main, sans agir sur les phalanges distales. Dans ce cas, les phalanges distales des doigts longs peuvent être mobiles par rapport aux phalanges moyennes à l'encontre de moyens élastiques de rappel. Cela permet de conférer une certaine résilience aux phalanges distales permettant leur adaptation souple aux objets saisis ou touchés par la main.

Selon une autre caractéristique avantageuse de l'invention, au moins une des phalanges des doigts longs comprend un capteur de sensibilité apte à délivrer un signal d'arrêt au(x) actionneur(s), le capteur étant avantageusement situé au niveau de la phalange moyenne. Il n'est cependant pas exclu d'équiper toutes les phalanges de tous les doigts longs de capteurs de sensibilité. Dès qu'un objet est saisi ou touché avec une certaine force de préhension, les capteurs de sensibilité délivrent un signal qui va être transmis aux actionneurs par l'intermédiaire d'un module électronique approprié afin de les stopper. Avantageusement, des moyens de stimulation sont situés au niveau de l'emboiture pour solliciter le moignon en réponse au signal d'arrêt généré par le capteur de sensibilité, de manière à reconstituer le toucher au niveau du moignon. Ces moyens de stimulation peuvent par exemple se présenter sous la forme d'un petit moteur apte à exercer une pression mécanique perceptible sur le moignon.

Selon un autre aspect intéressant de l'invention, l'index est pourvu de moyens de blocage en position étendue. Ainsi, l'index peut rester étendu alors que les trois autres doigts longs (majeur, annulaire et auriculaire) peuvent être repliés. On peut alors utiliser l'index tendu avec les autres doigts repliés dans de nombreuses situations, comme par exemple pour taper sur un clavier d'ordinateur, de téléphone, etc.

Selon un autre aspect de l'invention, les deux actionneurs peuvent être logés dans la paume de la main, avantageusement en amont de chaque paire de doigts longs. En raison de leur action aussi bien en flexion qu'en extension, les actionneurs peuvent présenter une puissance réduite, et ainsi un encombrement réduit permettant de les loger tous deux à l'intérieur de la paume de la main juste en amont des paires de doigts qu'ils actionnent respectivement.

En variante, les actionneurs peuvent être disposés au niveau de l'emboiture. Le positionnement des actionneurs est grandement dépendant de la configuration et de la taille du moignon.

Un principe de l'invention réside dans le fait d'utiliser des actionneurs qui agissent aussi bien en flexion qu'en extension sans utiliser de moyens de rappel élastiques, de manière à pouvoir réduire la taille des actionneurs pour pouvoir les loger dans la paume de la main. En raison de l'absence de moyens de rappel élastiques, les actionneurs n'ont pas besoin d'intégrer un frein pour bloquer les doigts dans une certaine position. Les câbles de flexion et d'extension agissent simultanément et positivement sans agir l'un contre l'autre. Lorsque les actionneurs sont arrêtés, les câbles de flexion et d'extension agissent tous les deux pour maintenir les doigts en position. Il faut bien garder à l'esprit que chaque actionneur agit simultanément sur un câble de flexion et un câble d'extension. Lorsque l'actionneur exerce une traction sur le câble de flexion, il relâche simultanément la traction sur le câble de flexion, et inversement. En pratique, des moyens de rattrapage de mou peuvent être prévus pour améliorer la traction et la détente des câbles de flexion et d'extension.

Un autre principe très intéressant de l'invention, qui pourrait être mis en oeuvre de manière totalement indépendante, réside dans le fait que les capteurs de sensibilité au niveau des doigts sont couplés à des moyens de stimulation au niveau de l'emboiture pour exercer une sollicitation mécanique ou électrique sur le moignon, permettant ainsi de reconstituer artificiellement la sensation de toucher au niveau des doigts.

Encore un autre principe de l'invention consiste à actionner uniquement les phalanges proximales et moyennes, les phalanges distales étant uniquement mobiles à l'encontre de moyens de rappel élastiques.

L'invention sera maintenant plus amplement décrite en référence aux dessins joints donnant à titre d'exemple non limitatif un mode de réalisation de l'invention.

Sur les figures :
La figure 1 est une vue schématique en plan d'une prothèse de main montée sur un moignon d'un avant-bras,
La figure 2 est une vue agrandie en coupe de la prothèse de main de la figure 1, et
Les figures 3 et 4 représentent de manière schématique deux modes de réalisation pour les actionneurs de la prothèse de main.

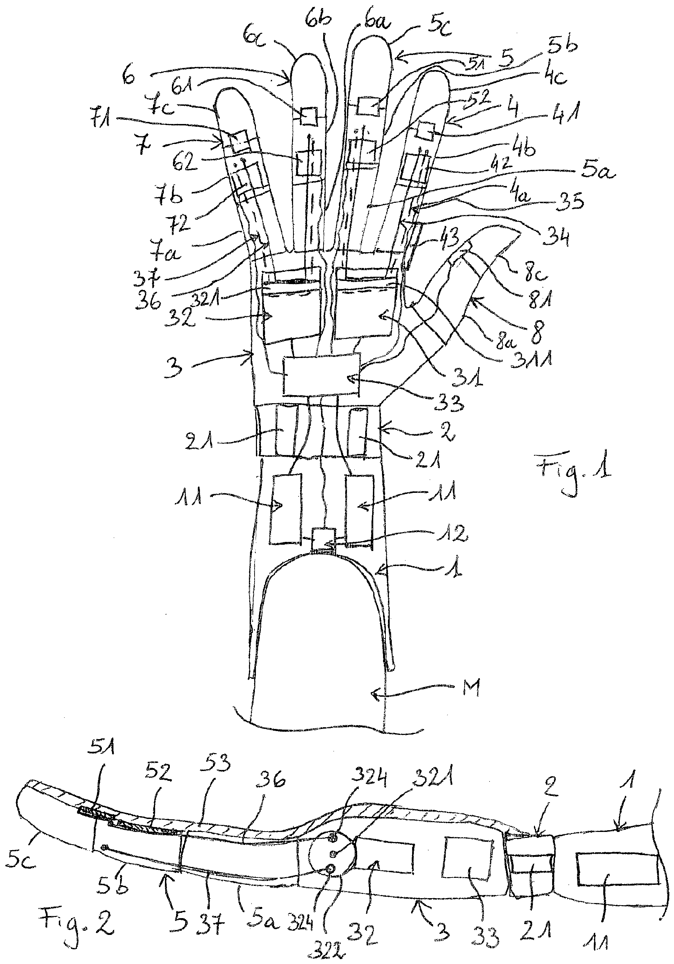

On se réfèrera tout d'abord aux figures 1 et 2 pour décrire de manière générale l'architecture et la structure d'une prothèse de main selon l'invention. La prothèse est destinée à être montée et fixée sur un moignon de membre supérieur par des moyens de fixation connus. Le moignon peut être situé au niveau du poignet, de l'avant bras, au niveau du coude, ou encore au-dessus du coude.

La prothèse de main ou main artificielle comprend cinq parties essentielles, à savoir une emboiture 1 destinée à être montée sur et autour d'un moignon M, un poignet 2 qui peut être rotatif et articulé, une paume de main 3, quatre doigts longs, à savoir un index 4, un majeur 5, un annulaire 6 et un auriculaire 7, ainsi qu'un pouce 8. En fonction de la position du moignon M, l'emboiture est reliée au poignet 2 avec une partie de liaison plus ou moins longue. Le poignet 2, la paume de main 3, les quatre doigts longs 4, 5, 6 et 7, ainsi que le pouce 8 présentent une configuration générale semblable ou sensiblement identique à une main réelle. Chaque doigt long comprend trois phalanges, à savoir une phalange proximale reliée à la paume 3, une phalange moyenne et une phalange distale formant le bout du doigt. Plus en détails, l'index 4 comprend une phalange proximale 4a reliée à la paume 3, une phalange moyenne 4b et une phalange distale 4c. Le majeur 5 comprend une phalange proximale 5a reliée à la paume 3, une phalange moyenne 5b et une phalange distale 5c. L'annulaire 6 comprend également une phalange proximale 6a, une phalange moyenne 6b et une phalange distale 6c. Enfin, l'auriculaire 7 comprend une phalange proximale 7a, une phalange moyenne 7b et une phalange distale 7c. Quant au pouce 8, il comprend une phalange proximale 8a et une phalange distale 8c. Toutes les phalanges des doigts longs 4, 5, 6 et 7 sont articulées les unes par rapport aux autres et par rapport à la paume de main 3, tout comme une main naturelle. Le pouce 8 est articulé au niveau de la liaison de la phalange proximale 8a avec la paume de main 3, et éventuellement au niveau de la jonction des deux phalanges 8a, 8c. Le pouce 8 peut être actionné au moyen d'un moteur approprié, ou en variante, le pouce est déplaçable manuellement sans l'intervention d'un moteur ou d'un mécanisme approprié.

L'emboiture 1, en plus de ses moyens de fixation au moignon M, peut servir de logement pour une ou plusieurs batteries 11 qui sont de préférence rechargeables. Optionnellement, l'emboiture comprend également des moyens de stimulation 12, qui peuvent par exemple être des moyens de pression mécanique ou d'impulsion électrique, aptes à exercer une sollicitation (pression mécanique ou impulsion électrique) sur le moignon M. De ce fait, au moins une partie des moyens de stimulation 12 doivent être situés au niveau du logement de l'emboiture 1 qui accueille le moignon M. Nous verrons ci-après quelle est la fonction et le fonctionnement de ces moyens de stimulation 12.

Le poignet 2 est ici relié à l'emboiture 1 et peut comprendre des moyens de rotation et/ou de flexion. Par exemple, le poignet 2 peut être pourvu d'une ou de plusieurs lames de ressorts élastiques 21 permettant une flexion résiliente. Le poignet 2 sert également au passage de câbles divers reliant les organes internes de l'emboiture 1 à la paume 3 et aux doigts.

La paume de main 3 est reliée en amont au poignet 2, en aval aux doigts longs 4, 5, 6, 7 et latéralement au pouce 8. La paume 3 peut servir de logement à un module électronique 33 qui va gérer le fonctionnement électrique des divers organes de la prothèse de main. La paume de main 3 peut également servir de logement à deux actionneurs 31 et 32 qui agissent sur les doigts longs 4, 5, 6 et 7 pour les mouvoir en flexion et en extension. En variante, il est également possible de loger les deux actionneurs 31 et 32 dans l'emboiture 1, de préférence à proximité du moignon M.

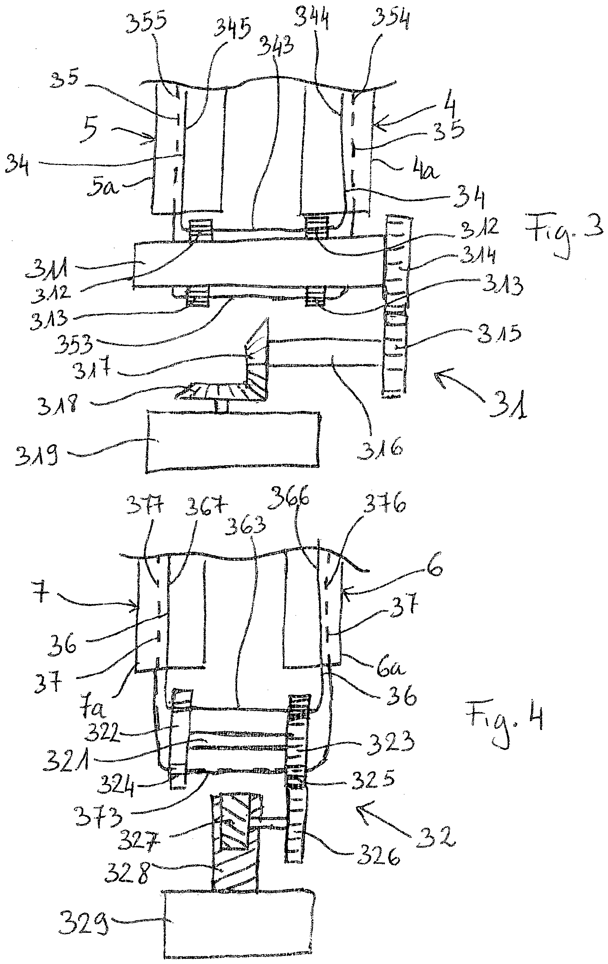

Selon l'invention, l'actionneur 31 agit sur l'index 4 et le majeur 5 en flexion et en extension, alors que l'actionneur 32 agit de même sur l'annulaire 6 et l'auriculaire 7. Les deux actionneurs 31 et 32 peuvent être identiques, ou encore différents, comme on le verra ci-après en référence aux figures 3 et 4. Sans trop rentrer dans les détails des actionneurs, on peut dire qu'ils comprennent chacun un arbre rotatif 311, 321 qui peut être entraîné en rotation au moyen d'un moteur par l'intermédiaire d'engrenages appropriés. Chaque arbre rotatif 311, 321, de par leur rotation, permet d'exercer une traction sur un câble de flexion et sur un câble d'extension. Plus précisément, en référence à l'actionneur 32 qui est également visible sur la figure 2, un câble de flexion 36 en forme de U s'étend sur la face intérieure de l'annulaire 6 et de l'auriculaire 7 en passant à travers des trous de passage 324 formés sur l'arbre rotatif 321. Le câble de flexion 36 s'étend ainsi de manière continue de l'annulaire 6 à l'auriculaire 7 an passant par l'arbre rotatif 321. Bien que cela ne soit pas représenté, le câble de flexion 36 passe aussi à travers d'autres trous de passage formés au niveau de la face intérieure des phalanges des doigts. Dans une forme de réalisation pratique représentée sur les figures, les deux extrémités du câble de flexion 36 sont fixés respectivement au niveau de la face intérieure des phalanges moyennes 6b et 7b. En d'autres termes, les câbles de flexion 36 ne s'étendent pas jusqu'au niveau des phalanges distales 6c et 7c. D'autre part, un câble d'extension 37 s'étend sensiblement de la même manière que le câble de flexion 36 sur la face extérieure de l'annulaire 6 et de l'auriculaire 7. C'est pourquoi il a été représenté en traits pointillés sur la figure 1. Tout comme le câble de flexion 36, le câble d'extension 37 s'étend de manière continue de l'annulaire 6 jusqu'à l'auriculaire 7 en passant par des trous de passage 324 prévu au niveau de l'arbre rotatif 321. Bien que non représenté, le câble d'extension 37 peut aussi passer à travers des trous de passage prévus au niveau de la face extérieure des phalanges de l'annulaire 6 et de l'auriculaire 7. Tout comme le câble de flexion 36, le câble d'extension 37 est fixé à ses extrémités sur la face extérieure des phalanges moyennes 6b et 7b de l'annulaire et de l'auriculaire.

Ainsi, en entrainant en rotation l'arbre rotatif 321, une traction est exercée sur un des câbles 36, 37 de manière à entrainer l'annulaire 6 et l'auriculaire 7 en flexion et/ou en extension. Lorsque l'arbre 321 tourne dans un sens, le câble de flexion est par exemple mis sous tension, alors que le câble d'extension est relâché. Inversement, lorsque l'arbre 321 tourne dans l'autre sens, le câble de flexion est relâché et le câble d'extension est mis sous tension.

Il faut remarquer qu'il n'y a aucun moyen de rappel élastique qui agit à l'encontre de la traction exercée par l'actionneur. De ce fait, il n'est pas nécessaire de prévoir un frein au niveau de l'actionneur pour bloquer les doigts dans une position donnée. L'arrêt de l'actionneur suffit à maintenir les doigts en position.

Selon une autre caractéristique, les deux câbles de flexion 36 et d'extension 37 en forme de U passent librement sans blocage et avec un faible frottement à travers les trous de passage 324 de l'arbre rotatif 321, de sorte qu'il se crée automatiquement un équilibrage ou une répartition des forces entre l'annulaire et l'auriculaire. Si l'annulaire vient en butée contre un objet, l'actionneur continue à exercer une traction jusqu'à ce que l'auriculaire vienne lui également en butée, par exemple contre cet objet. Au final, les deux doigts sont en butée contre cet objet, et la force de préhension est répartie de manière équilibrée entre les deux doigts. Bien que non représenté, on peut prévoir des moyens de rattrapage ou de mise à disposition de mou au niveau des câbles pour assurer une tension minimum.

Ce qui vient décrit en référence à l'actionneur 32 et aux deux doigts 6 et 7 est également valable pour l'actionneur 31 et les deux doigts 4 et 5. De ce fait, chaque paire de doigts longs (index/majeur et annulaire/auriculaire) sont déplacées en flexion et en extension au moyen d'un actionneur dédié et par l'intermédiaire de deux câbles de flexion et d'extension en forme de U qui s'étendent de manière continue sur les faces intérieure respectivement extérieure des doigts.

Les extrémités des câbles pourraient s'étendre jusqu'au niveau des phalange distales des doigts longs, mais de préférence, les extrémités sont reliés aux phalanges moyennes, et on prévoit alors des moyens de rappel élastiques 41, 51, 61 et 71 au niveau de l'articulation entre les phalanges moyennes et les phalanges distales pour permettre un déplacement résilient des phalanges distales. Les moyens de rappel élastiques 41, 51, 61 et 71 peuvent par exemple se présenter sous la forme d'un ressort classique ou encore d'une lame élastique. De tels moyens de rappel élastiques peuvent également être prévus au niveau de la jonction des phalanges du pouce 8, comme indiqué en 81.

Selon une autre caractéristique de l'invention, on peut prévoir des moyens de blocage 43 pour bloquer l'index 4 en position étendue ou semi-étendue, permettant ainsi de se servir de l'index pour des applications particulières, comme par exemple pour taper sur un clavier. Les moyens de blocage 43 peuvent très simplement se présenter sous la forme d'une broche qui bloque la phalange proximale 4a par rapport à la paume de main 3.

Selon un autre aspect de l'invention, au moins une partie de la paume 3 et des doigts, notamment la face interne de la main, peut être recouverte d'un matériau souple et mou, reproduisant sensiblement la texture de la peau.

Selon une autre caractéristique, au moins certaines phalanges des doigts peuvent être pourvues de capteurs de sensibilité 42, 52, 62, 72 aptes à délivrer un signal au module électronique 33 pour arrêter le fonctionnement des actionneurs 31 et 32. Les capteurs de sensibilité peuvent par exemple être prévus au niveau de la face intérieure des phalanges moyennes 4b, 5b, 6b et 7b. On peut également en prévoir au niveau des autres phalanges, et même au niveau du pouce. Le signal d'arrêt délivré par les capteurs peut également servir à commander les moyens de stimulation 12 prévus au niveau du moignon M. Ainsi, dès qu'un ou plusieurs des capteurs de sensibilité viennent en contact suffisant avec un objet, les actionneurs 31 et 32 sont arrêtés et les moyens de stimulation 12 sont activés de manière à générer une sollicitation au niveau du moignon M. Le porteur de la prothèse de main perçoit alors directement l'information selon laquelle un ou plusieurs doigts de la main est en contact de préhension avec un objet. Il est même possible de prévoir des moyens de stimulation 12 qui génèrent une sollicitation distincte pour chaque doigt de la main. De cette manière, le porteur de la prothèse de main perçoit immédiatement quel doigt est en contact de préhension avec un objet.

On se référera maintenant à la figure 3 pour décrire un mode de réalisation de l'actionneur 31. Cet actionneur est disposé dans la paume de main 3 directement en amont de l'index 4 et du majeur 5. L'actionneur 31 comprend un arbre rotatif 311 pourvu de quatre trous ou oeillets de passage 312 et 313. Le câble de flexion 34 en forme de U comprend une section 344 qui s'étend le long de la face intérieure des phalanges de l'index 4 et une section symétrique 345 qui s'étend le long de la face intérieure des phalanges du majeur 5. Ces deux sections 344 et 345 sont reliées ensemble par une section de liaison 343 qui passe librement à travers les trous ou oeillets de passage 312 de l'arbre 311. De manière symétrique, le câble d'extension 35, qui est représenté en pointillés, comprend une section 354 qui s'étend le long de la face extérieure des phalanges de l'index 4, une autre section 355 qui s'étend le long de la face extérieure des phalanges du majeur 5, ainsi qu'une section de liaison 353 qui passe librement à travers les deux trous ou oeillets de passage 313 de l'arbre 311. Les trous de passage 312 et 313 ont été représentés de manière diamétralement opposée sur la figure 3, mais on peut également imaginer qu'ils soient disposés les uns à côté des autres. Pour entrainer l'arbre rotatif 311 en rotation, celui-ci est pourvu d'une roue dentée 314 qui est en prise avec une autre roue dentée 315 reliée par un arbre 316 à un engrenage conique 317 en prise avec un autre engrenage tronconique perpendiculaire 318 entrainé par un moteur 319. Tous les éléments constitutifs de l'actionneur 31 peuvent être logés dans un boitier de taille très réduite susceptible d'être reçu à l'intérieur de la paume de main 3. Pour bloquer l'index et le majeur dans une position souhaitée, le simple arrêt du moteur 19 suffit sans nécessité de frein. Il est à noter que les deux câbles de flexion 34 et d'extension 35 sont mis sous tension ou relâchés par un arbre rotatif unique 311.

En se référant à la figure 4, on voit un mode de réalisation pratique pour l'actionneur 32 qui agit sur l'annulaire 6 et l'auriculaire 7. L'arbre rotatif 321 est pourvu de deux flasques 322 et 323 qui sont chacun percés de deux trous de passage 324 et 325. Les trous peuvent être disposés de manière diamétralement opposée ou encore de manière plus rapprochée. Le câble de flexion 36 comprend une section 366 qui s'étend sur la face intérieure de l'index 6 et une section 367 qui s'étend sur la face intérieure de l'auriculaire 7, ces deux sections étant reliées ensemble par une section de liaison 363 qui passe à travers les deux trous formés dans les deux flasques 322 et 323. De manière symétrique, le câble d'extension 37 comprend une section 376 qui s'étend sur la face extérieure de l'annulaire 6 et une section 377 qui s'étend sur la face extérieure de l'auriculaire 7, ainsi qu'une section de liaison 373 qui passe à travers les deux trous 324 et 325 des deux flasques 322 et 323. Il est à noter que les câbles 36 et 37 passent librement avec possibilité de coulissement à travers les trous de passage 324 et 325. Pour entrainer l'arbre rotatif 321 en rotation, un des deux flasques 323 dentés est en prise avec une roue dentée 326 reliée par un arbre à une roue dentée inclinée 327 en prise avec une vis sans fin 328 entrainée en rotation par un moteur 329. En actionnant ce moteur 329, l'arbre rotatif 321 est entrainé en rotation et un des deux câbles 36, 37 est mis sous tension de manière à fléchir les doigts ou à les étendre. Il faut noter que tous les éléments constitutifs de l'actionneur 32 peuvent être logés dans un boitier de taille très réduite pouvant être inséré aisément à l'intérieur de la paume de main 3.

Comme susmentionné, les deux actionneurs 31 et 32 peuvent être identiques ou différents. Un moteur similaire peut également être prévu pour le pouce 8.

Grâce à l'invention, on dispose d'une prothèse de main dont les doigts longs sont actionnés par paires en flexion et en extension à l'aide de deux actionneurs, avantageusement sans frein qui sont logés à l'intérieur de la paume de main. Les moyens de stimulation au niveau du moignon couplés aux capteurs de sensibilité constituent une caractéristique qui peut être mise en oeuvre indépendamment de l'actionnement des doigts par paires en flexion et en extension.

## Revendications

1. Prothèse de main destinée à être montée sur un moignon (M) d'un membre supérieur, la prothèse de main comprenant une emboiture (1) à monter sur le moignon (M), une paume de main (3), quatre doigts longs (4,5,6,7) et un pouce (8), les quatre doigts longs (4,5,6,7) comprenant chacun trois phalanges mobiles (4a, 4b, 4c,5a, 5b, 5c, 6a, 6b, 6c, 7a, 7b, 7c) articulées les unes par rapport aux autres et par rapport à la paume (3),la prothèse de main comprenant en outre des moyens d'actionnement (31,32,34,35,36,37) pour déplacer au moins deux des phalanges de chaque doigt long, les moyens d'actionnement comprenant deux actionneurs distincts (31, 32) actionnant chacun une paire de doigts longs (4, 5, 6, 7) en flexion et en extension, **caractérisée en ce que** chaque paire de doigts longs (4,5,6,7) comprend un câble de flexion et (34,36) et un câble d'extension (35,37) communs à ladite paire de doigts longs, chaque câble de flexion et d'extension s'étendant sensiblement en forme de U sur les deux doigts de la paire de doigts longs, les deux câbles de flexion et d'extension (34, 36, 35, 37) étant sollicités en traction par un actionneur unique respectif (31, 32), permettant une répartition équilibrée des forces de traction sur la paire de doigts longs.

2. Prothèse de main selon la revendication 1, dans laquelle chaque actionneur (31, 32) comprend un arbre rotatif (311, 321) disposé sensiblement perpendiculairement à la direction longitudinale des doigs longs (4, 5, 6, 7), l'arbre (311, 321) comprenant au moins deux trous de passage (312, 313,324,325) respectivement pour le câble de flexion (34, 36) et le câble d'extension (35, 37), ces derniers étant aptes à coulisser à travers les trous de passage (312, 313, 324, 325) pour équilibrer les forces de traction sur la paire de doigts longs.

3. Prothèse de main selon l'une quelconque des revendications precédentes, dans laquelle les actionneurs (31, 32) sont aptes à actionner les phalanges proximales (4a, 5a, 6a, 7a,) et moyennes (4b, 5b, 6b,7b,) des doigts longs.

4. Prothèse de main selon l'une quelconque des revendications précédentes, dans laquelle les phalanges distales (4c, 5c, 6,c, 7c) des doigts longs sont mobiles par rapport aux phalanges distales (4b, 5b, 6b, 7b) à l'encontre de moyens élastiques de rappel (41, 51, 61, 71).

5. Prothèse de main selon l'une quelconque des revendications précédentes, dans laquelle au moins une des phalanges des doigts longs (4, 5, 6, 7) comprend un capteur de sensibilité (42, 52, 62, 72) apte à délivrer un signal d'arrêt au(x) actionneur(s) (31, 32), le capteur étant situé par exemple au niveau de la phalange moyenne (45b, 5b, 6b, 7b).

6. Prothèse de main selon la revendication 5, dans laquelle des moyens de stimulation (12) sont situés au niveau de l'emboiture (1) pour solliciter le moignon (M) en réponse au signal d'arrêt généré par le capteur de sensibilité (42, 52, 62, 72) de manière à reconstituer le toucher au niveau du moignon.

7. Prothèse de main selon l'une quelconque des revendications pécédentes, dans laquelle l'index (4) est pourvu de moyens de blocage (43) en position étendue.

8. Prothèse de main selon l'une des quelconques des precedents, dans laquelle les deux actionneurs (31,32) sont logés dans la paume de la main (3), en amont de chaque paire de doigts longs (4, 5, 6, 7).

9. Prothèse de main selon l'une quelconque des revendications 1 à 7, dans laquelle les actionneurs (31, 32) sont disposés au niveau de l'emboiture (1).

## Patentansprüche

1. Handprothese is dazu bestimmt auf einem Stumpf (M) einer oberen Extremität angebracht zu werden, die Handprothese umfasst einen Sockel (1) zur Befestigung auf den Stumpf (M), eine Handfläche (3) vier lange Finger (4,5,6,7) und einen Daumen (8), die vier langen Finger (4,5,6,7) mit jeweils drei beweglichen Phalangen (4a, 4b, 4c, 5a, 5b, 5c, 6a, 6b, 6c, 7a, 7b, 7c) zueinander und relativ zu der Handfläche (3), die Handprothese umfassend außerdem ein Betätigungsmittel (31,32,34,35,36,37) für mindestens zwei Phalangen ,um mindestens zwei Phalangen in jedem langen Finger zu bewegen, die Betätigungsmittel bestehen aus zwei separaten Betätigunsmitteln (31, 32) jeweils ein Paar von langen Fingern (4, 5, 6, 7) Beugung und Dehnung, **dadurch gekennzeichnet, dass** jedes Paar der langen Finger (4,5,6,7) ein Beugungskabel (34,36) und einen gemeinsamen Dehnungskabel (35,37) hat im besagten Paar der langen Finger, jedes Beugungs- und Dehungskabel erstreckt sich im Wesentlichen U-förmig auf beiden Fingern des Paares von langen Fingern, die Beugungs- und Dehungskabel (34, 36, 35, 37) sind in der Spannung durch einen entsprechenden einzelnen Aktuator geladen (31, 32), was eine ausgeglichene Verteilung der Zugkräfte auf das Paar von langen Fingern ermöglicht.

2. Handprothese nach Anspruch 1, wobei jeder Aktuator (31, 32) eine Drehwelle (311, 321), angeordnet im Wesentlichen senkrecht zur Längsrichtung der langen Finger (4, 5, 6, 7), die Achse (311, 321) umfasst mindestens zwei Durchgangslöcher (312, 313, 324, 325) jeweils an der Beugungslinie (34, 36) und dem Dehungskabel (35, 37), letztere können durch die Durchgangslöcher gleiten (312, 313, 324, 325), um die Zugkräfte auf das Paar von langen Fingern auszubalancieren.

3. Handprothese nach einem der vorhergehenden Ansprüche, wobei die Aktuatoren (31, 32) angepasst sind, die proximalen Phalangen zu betätigen (4a, 5a, 6a, 7a) und mittleren (4b, 5b, 6b, 7b) langen Fingern.

4. Handprothese nach einem der vorhergehenden Ansprüche, wobei die distalen Phalangen (4c, 5c, 6c, 7c) der langen Finger beweglich sind relativ zu den distalen Phalangen (4b, 5b, 6b, 7b) gegenüber elastische Rückstellmittel (41,51,61,71).

5. Handprothese nach einem der vorhergehenden Ansprüche, wobei mindestens eine der Phalangen der langen Finger (4, 5, 6, 7) mit einen Empfindlichkeitssensor (42, 52, 62, 72) angepasst ist, um ein Signal Stopp bei (x) Aktuator(en) (31, 32) auszugeben, wobei der Sensor beispielsweise an der mittleren Phalanx (45b, 5b, 6b, 7b) angebracht ist.

6. Handprothese nach Anspruch 5, wobei die Stimulationsmittel(12) am Sockelelement (1) angeordnet zum Vorspannen des Stumpfes (M), das als Reaktion auf das Stoppsignal des Empfindlichkeit Sensors erzeugt wird (42, 52, 62, 72) so wie die Berührung am Stumpf rekonstruiert.

7. Handprothese nach den vorhergehenden Ansprüchen, wobei der Index (4) mit Verriegelungsmitteln(43) in ausgefahrener Position ausgestattet ist.

8. Handprothese nach einem der vorhergehenden Ansprüche, wobei die beiden Aktuatoren (31,32) in der Handfläche (3), auf der Oberseite von jedem Paar von langen Fingern (4, 5, 6, 7) liegen.

9. Handprothese nach einem der Ansprüche 1 bis 7, wobei die Aktuatoren (31, 32) an dem Sockel (1) angeordnet sind.

## Claims

1. Hand prosthesis intended to be mounted on the stump (M) of one of the upper limbs, the hand prosthesis is comprised of a socket (1) to be mounted on the stump (M), a hand palm (3), four (4,5,6,7) fingers and a thumb (8), the four fingers (4,5,6,7) are each comprised of three mobile phalanges (4A, 4B, 4 c, 5A, 5B, 5 c, 6a, 6B, 6 c (, 7a, 7b, 7 c) each related to the other and in relation with the palm (3), in addition, the prosthetic hand possesses means of actuation (31,32,34,35,36,37) to move at least two phalanges of each finger, the means of actuation being comprised of two separate actuators (31, 32) each operating a pair of fingers (4, 5, 6, 7) for flexion and extension **characterised in that** each pair of fingers (4,5,6,7) includes a flexion cable and (34.36) and an extension cable (35,37) common to the pair of fingers, each flexion and extension cable extend substantially to form a U shape on the two fingers of the pair of fingers, the two flexion and extension cables being (34, 36, 35 (37) tension loaded by a single respective actuator (31, 32), allowing a balanced distribution of traction forces on the pair of fingers.

2. Hand prosthesis according to claim 1, wherein each actuator (31, 32) is comprised of a rotary shaft (311, 321) disposed substantially perpendicular to the longitudinal direction of the fingers (4, 5, 6, 7), the shaft (311, 321) comprised of at least two through holes (312, 313,324,325) respective to the flexion cable (34, 36) and the extension cable (35, 37), the latter being able to slide through the through holes (312, 313, 324, 325) to balance the traction forces on the pair of fingers.

3. Hand prosthesis according to any preceding claim, wherein the actuators (31, 32) are able to actuate the proximal (4a, 5a, 6a, 7a) and middle (4b, 5b, 6b, 7b) phalanges of the fingers.

4. Hand prosthesis according to any preceding claim, wherein the distal phalanges (4c, 5c, 6 c, 7 c) of the fingers are mobile relative to the distal phalanges (4b, 5b, 6b, 7b) in respect to the elastic return means. (41, 51, 61, 71).

5. Hand prosthesis according to any preceding claim, wherein at least one of the phalanges of the fingers (4, 5, 6, 7) comprises a sensitivity sensor (42, 52, 62, 72) adapted to outputting a stop signal to the (x) actuator (s) (31, 32), the sensor being located for example at the middle phalanx (45b, 5b, 6b, 7b).

6. Hand prosthesis according to claim 5, in which the means of stimulation (12) are located at the socket (1) to solicit the stump (M) to react to the stop signal generated by the sensitivity sensor ( 42, 52, 62, 72) so as to reconstruct touch at the stump level.

7. Hand prosthesis according to any one of the preceding claims, wherein the index (4) is provided with locking means (43) in extended position.

8. Hand prosthesis according to any preceding claim, wherein the two actuators (31,32) are housed in the palm of the hand (3), upstream of each pair of fingers (4, 5, 6 7).

9. Hand prosthesis according to any one of claims 1 to 7, wherein the actuators (31, 32) are arranged at the socket (1).
